## Europäisches Patentamt
## European Patent Office
## Office européen des brevets

(11) Veröffentlichungsnummer: **0 209 724 B1**

# EUROPÄISCHE PATENTSCHRIFT

(45) Veröffentlichungstag der Patentschrift: 25.09.91

(21) Anmeldenummer: 86108265.9

(22) Anmeldetag: 18.06.86

(51) Int. Cl.⁵: **A61K 35/78, A61K 7/48,**
//(A61K35/78,31:71)

---

(54) **Präparat mit Wirkung gegen Akne sowie Verwendung von Wirkstoffextrakt des Queckenweizens.**

---

(30) Priorität: 18.06.85 HU 238985

(43) Veröffentlichungstag der Anmeldung:
28.01.87 Patentblatt 87/05

(45) Bekanntmachung des Hinweises auf die
Patenterteilung:
25.09.91 Patentblatt 91/39

(84) Benannte Vertragsstaaten:
**AT BE CH DE FR GB IT LI LU NL SE**

(56) Entgegenhaltungen:
**DE-A- 2 922 467**
**FR-A- 1 367 618**
**GB-A- 2 090 135**

(73) Patentinhaber: **BIOGAL GYOGYSZERGYAR**
**Pallagi ut 13**
**H-4042 Debrecen(HU)**

(72) Erfinder: **Kekesi, Sandor**
**Aprilis 4. u. 23/b**
**H-4029 Debrecen(HU)**
Erfinder: **Tamási, Piroska, Dr.**
**Bem tér 24**
**H-4029 Debrecen(HU)**

Erfinder: **Pál, Veronika**
**Hatvan u. 1/c**
**H-4029 Debrecen(HU)**
Erfinder: **Kristof Szvitil, Ilona**
**Németh L. u. 24**
**H-4029 Debrecen(HU)**
Erfinder: **Jancs , Sándor, Dr.**
**Kardos u. 28**
**H-4042 Debrecen(HU)**
Erfinder: **Bacsa, György, Dr.**
**Csap u. 90**
**H-4042 Debrecen(HU)**
Erfinder: **Kovács Hadady, Katalin, Dr.**
**Lehel u. 14**
**H-4024 Debrecen(HU)**

(74) Vertreter: **Hoffmann, Klaus, Dr. rer. nat. et al**
**Hoffmann . Eitle & Partner Patentanwälte**
**Postfach 81 04 20 Arabellastrasse 4**
**W-8000 München 81(DE)**

---

## Beschreibung

Die Erfindung betrifft ein Präparat mit Wirkung gegen Akne sowie die Verwendung von Wirkstoffextrakt des Queckenweizens. Die Erfindung betrifft demnach ein zur Heilung der am häufigsten auftretenden Typen von primär durch den Stoffwechsel verursachten krankhaften Hautveränderungen, der Seborrhoe, insbesondere der unterschiedlichen Akneformen (Mitesser, Pickel).

Die Seborrhoe ist ein auf einer gesteigerten und auch qualitativ veränderten Tätigkeit der Talgdrüsen der Haut beruhender Krankheitsprozeß, der sich in einer gesteigerten Produktion von Hauttalg und in dessen veränderter Zusammensetzung manifestiert. Durch die erhöhte Talgproduktion wird die Haut fettglänzend, Parakeratose und unterschiedlichen Schälformen treten auf. Die Seborrhoe hat aber auch Formen, wo außer den aufgezählten Symptomen auch eine Hyperkerotose der Haarbalgkanäle und ihre Verstopfung mit dickgewordenem Talg vorkommen kann. Diese Erscheinung nennt man Komedonenbildung , sie tritt vor allem auf der Gesichtshaut und in den Arnozan'schen Dreiecken auf.

Nach den meisten der gegenwärtig bestehenden Vorstellungen hängt die Entstehung der Seborrhoe mit dem endokrinen System zusammen, die bakteriellen Infektionen sind schon ihre charakteristischen Folgen.

Die Akne ist eine mehr oder weniger schwere Manifestation der zur Zeit der Puberität eintretenden Seborrhoe.

Zu Beginn sind die fettglänzenden Komedonen wahrzunehmen. Als spezielles Symptom der in Jugendalter auftretenden Akne konnte eine im allgemeinen anwesende Bakteriumart, des Corynebacterium acnes, identifiziert werden. Zur symptomatischen Behandlung der von dieser Bakterienart verursachten Veränderungen ist in der ungarischen Patentschrift Nr. 171 517 ein oral applizierbares Präparat beschrieben, das inaktivierte Stämme von Corynebacterium acnes in lyophilisierter Form enthält. Das in der Patentschrift vorgeschlagene Mittel beruht auf aktiver Immunität.

Die an unterschiedlichen Formen von Akne leidenden jungen Menschen waren auch früher schon wegen der groben und augenfälligen Entstellung ihres Gesichtes sozial benachteiligt, deshalb wurden zahlreiche Verfahren zur Heilung dieser Krankheit oder doch wenigstens ihrer Symptome entwickelt. Die schwächeren Formen der Acne vulgaris reagieren bei Einhaltung einer pflanzlichen, vitaminreichen, fett- und kohlehydratarmen Diät auf die entsprechende lokale Behandlung gut. In diesen Fällen wird dem Kranken häufig empfohlen, die kranken Hautstellen mit Schwefel, Ichthyol und gegebenenfalls Salicyl enthaltenden Präparaten wie Salben oder Schüttelmischungen zu behandeln, zusammen mit desinfizierend wirkenden lauwarmen Umschlägen. Schwefelhaltige Verbindungen, Cysteinderivate werden unter anderem in dén DT-AS 1 908 574, 1 918 907 und 2 620 850 sowie der US-PS 4 428 933 beschrieben. Das Präparat gemäß letzterer enthält neben allgemein bekannten schwefelhaltigen und Peroxydverbindungen auch Naturstoffe wie Senfkörner, Hafermehl, Hefe sowie Eigelb.

Hier soll nur auf die lokal anwendbaren Präparate eingegangen werden, auf die gegen Akne ausgearbeiteten oral applizierbaren Präparate jedoch nicht.

Die meisten der gegen Akne lokal angewendeten Präparate beruhen in ihrer Wirkung darauf, daß das in den Komedonen anwesende Corynebacterium acnes die anaeroben Bedingungen bevorzugt. Die Behandlung mit Azoverbindungen und Peroxyde enthaltenden Präparaten führt zu einem vorübergehenden Verschwinden der Symptome. In zahlreichen Fällen werden die lokal eine sauerstoffreiche Umgebung gewährleistenden Verbindungen mit antibakteriellen Mitteln kombiniert, wodurch oft Synergismus eintritt. Präparate dieser Art haben als Grundlage die Vorstellung, daß die Peroxyde die Bildung der extracellulären Lipase, die Antibiotika die Vermehrung des die Lipase produzierenden Bakteriums (C. acnes) verhindern. Derartige Präparate sind in den britischen Patentschriften Nr. 1 539 771, 2 088 717 und 2 090 135, der US-PS 4 446 145, der französischen Patentschrift Nr. 2 383 667, der DT-AS 2 918 943 und der PCT-Anmeldung 83/628 beschrieben.

Aus anderen Patentschriften sind Präparate bekannt, die als Wirkstoff primär Antibiotika enthalten. Zur Behandlung von Akne werden von den Antibiotika insbesondere Penicillin und Erythromycin (britische Patentschrift Nr. 1 587 428, US-PS 4 261 962) eingesetzt, jedoch ist auch die Verwendung von Tetracyclin, Neomycin (britische Patentschrift Nr. 1 054 124), Clindamycin, Lincomycin und Grisefulvin (DT-AS 2 557 431, europäische Patentanmeldungen 52.705 und 52.404) bekannt.

Schließlich wurden auch Cremes und Salben gegen Akne ausgearbeitet, die als Wirkstoff Pflanzenextrakte natürlicher Herkunft an sich oder in Kombination mit anderen Wirkstoffen, insbesondere Antibiotika, enthalten.

Das in der europäischen Patentanmeldung Nr. 41 030 beschriebene Präparat enthält die Alkaloide von Vincea rosea zusammen mit einem das Eindringen erleichternden Adjuvant. Gemäß der ungarischen Patentanmeldung Nr. 3213/82 werden als Wirkstoffe von Präparaten gegen Akne Drogen verwendet, die mit Obstgeist extrahiert wurden. Gleichzeitig enthält das Präparat schwefelhaltige Verbindungen (Ichthyol).

Bei der Anwendung der beschriebenen Präparate kommt es oft zu einer irritativen, lichtsensibilisierenden und in vielen Fällen allergisierenden Wirkung. Bei der Verwendung von schwefelhaltige Verbindungen oder energisch wirkende Peroxyde enthaltenden Präparaten ist auch das Schälen der Haut häufig, was eine Quelle für Neuinfektionen darstellt.

Ziel der Erfindung ist die Ausarbeitung eines Präparates, das das Krankheitsbild der Seborrhoe zum Verschwinden bringt, den Heilprozeß der Haut schnell ablaufen läßt und die bekannten Nebenwirkungen nicht hervorruft.

Grundlage der Erfindung ist die Erkenntnis, daß die fettige, das Krankheitsbild der Akne aufweisende Haut innerhalb kurzer Zeit symptomfrei gemacht werden kann, wenn man ein Präparat verwendet, welches die Haut beruhigt, gleichzeitig die Sekretion beschleunigt, die Haut desinfiziert und die Talgabsonderung allmählich normalisiert. Eine diese Wirkungen in sich vereinigende Krem war bisher nicht bekannt.

Es wurde nun gefunden, daß zur Erreichung des oben formulierten Ziels ein unterschiedliche pflanzliche Extrakte enthaltendes Präparat am besten geeignet ist. Die Versuche ergaben überraschenderweise, daß der Queckenweizen (Agropyron repens) in seinem Wurzelstock (Graminis rhizoma) Stoffe enthält, die - extrahiert und mit den in der kosmetischen Industrie allgemein verwendeten Träger- und Hilfsstoffen vermischt - erfolgreich zur Heilung der unterschiedlichen Erscheinungsformen der Akne herangezogen werden können. Neben dem Extrakt des Queckenweizens kann das Präparat noch die Extrakte anderer Pflanzen enthalten. Die auf die Haut ausgeübte normalisierende Wirkung ist auf den Gehalt an Inosit, Triticin, Saponin, Carotin, Kieselsäure, Apfelsäure, Citronensäure, Gerbstoffen, Vitaminen und Saccharid der Präparate zurückzuführen. Die Haut wird von dem Präparat nicht ausgetrocknet und nicht irritiert.

Die vorteilhafte Wirkung des Extraktes von Graminis rhizoma ist darauf zurückzuführen, daß der Extrakt eine verhältnismäßig große Menge Saponin enthält, welches in geringer Dosis die Sekretion anregt und die Resorption anderer Stoffe fördert. Der Saponingehalt des Präparates kann noch erhöht werden, indem man den Extrakt weiterer saponinhaltiger Drogen (zum Beispiel Equiseti herba) zusetzt.

Zur Bekämpfung der eine Begleiterscheinung der unterschiedlichen Formen der Akne bildenden bakteriellen Infektion können die erfindungsgemäßen, pflanzliche Extrakte enthaltenden Präparate noch zusätzlich Antibiotika enthalten. Diese Präparate bringen auch im Falle schwererer Formen der Akne innerhalb verhältnismäßig kurzer Zeit eine Besserung. Der Extrakt von Graminis rhizome und das Antibiotikum ergänzen sich gut.

Die Erfindung betrifft ein Präparat mit Wirkung gegen die verschiedenen Formen der Akne, insbesondere in Form von Creme, Tonikum, Seife, Körperpflegelotionen, Gesichtspackungen und Shampoos, enthaltend 2-95 Gewichtsprozent saponin-haltigen Wirkstoffextrakt aus dem Wurzelstock (Graminis rhizoma) des Queckenweizens (Agropyron repens) und 5-98 Gewichtsprozent der in der Kosmetik im allgemeinen üblichen Zusätze und/oder Verdünnungsmittel, vorzugsweise natürliche Fette, Öle, Emulgiermittel, Konservierungsmittel, Duftstoffe und gegebenenfalls ein Antibiotikum und/oder weitere Pflanzenextrakte.

Als Antibiotikum können zum Beispiel Penicillin, Erythromycin, Oxytetracyclin oder Neomycin verwendet werden. Erythromycin ist bevorzugt.

Die erfindungsgemäßen Präparate können verschiedene Formen haben, zum Beispiel kann die Formulierung der Extrakte zu Krems, Gesichtstonik, Seife, Körperlotion, Gesichtsmilch, Shampoon, Gelee-Gesichtspackungen erfolgen.

Die Erfindung wird im folgenden an Hand von Beispielen näher erläutert. Die Beispiele 1-14 zeigen die Herstellung der pflanzlichen Extrakte, die weiteren Beispiele veranschaulichen die Formulierung dieser Extrakte zu anwendbaren Präparatformulierungen.

Beispiel 1

Herstellung eines wäßrigen Extraktes des Wurzelstockes (Graminis rhizoma) des Queckenweizens (Agropyron repens)

10 kg gereinigte und zerschrotete Wurzelstöcke werden in 100 Liter Wasser der Temperatur von 95 °C 2 Stunden lang gerührt. Dann wird das Gemisch zentrifugiert. Der erhaltene Extrakt wird bis zur weiteren Verwendung an einem kühlen Ort, vor Licht geschützt und von Luft abgeschlossen verwahrt.

Beispiel 2

Herstellung eines alkoholischen Extraktes des Wurzelstockes (Graminis rhizoma) des Queckenweizens (Agropyron repens)

10 kg gereinigte und zerschrotete Wurzelstöcke werden in 60 Liter 70 %igen Äthanols eingerührt. Man läßt das Gemisch 5 Tage lang stehen, es wird täglich einigemale aufgerührt. Dann wird das Gemisch filtriert, und das Filtrat bis zur weiteren Verwendung an einem kühlen Ort, vor Licht geschützt und von Luft abgeschlossen verwahrt.

Beispiel 3

Herstellung eines wäßrigen Extraktes aus den oberirdischen Trieben (Equiseta herba) des Schachtelhalms (Equisetum arvense L.)

10 kg gereinigte und zerkleinerte Schachtelhalmtriebe werden 20 Minuten lang in 100 Liter Wasser der Temperatur 90 °C gerührt. Dann wird das Gemisch filtriert. Das Filtrat wird bis zur weiteren Verwendung vor Wärme, Licht und Luft geschützt aufbewahrt.

Beispiel 4

Herstellung eines alkoholischen Extraktes aus den oberirdischen Trieben (Equiseta herba) des Schachtelhalms (Equisetum arvense L.)

Man arbeitet auf die im Beispiel 2 beschriebene Weise, geht jedoch von Schachtelhalmtrieben aus.

Beispiel 5

Herstellung eines wäßrigen Extraktes der Scheinfrüchte (Cynosbati pseudofructus) der Heckenrose (Rosa canina)

10 kg getrocknete und zerkleinerte Hagebutten werden 45 Minuten lang in 60 °C warmem Wasser eingeweicht. Dann wird das Gemisch abfiltriert, mit einem Konservierungsmittel versetzt und schließlich bis zur weiteren Verwendung vor Wärme, Licht und Luft abgeschlossen aufbewahrt.

Beispiel 6

Herstellung eines alkoholischen Extraktes der Scheinfrüchte (Cynosbati pseudofructus) der Heckenrose (Rosa canina)

Man arbeitet auf die im Beispiel 2 beschriebene Weise.

Beispiel 7

Herstellung eines wäßrigen Auszuges von Blüten (Chamomillae flos) der Kamille (Matricaria)

10 kg getrocknete, gereinigte Kamillenblüten werden in 80 Liter Wasser der Temperatur von 80 °C 10 Minuten lang gerührt. Dann wird das Gemisch abfiltriert. Das Filtrat wird bis zur weiteren Verwendung vor Wärme, Licht und Luft geschützt aufbewahrt.

Beispiel 8

Herstellung eines alkoholischen Auszuges von Blüten (Chamomillae flos) der Kamille (Matricaria)

Man arbeitet gemäß Ph. Hg. Vi.

Beispiel 9

Herstellung eines wäßrigen Auszuges von Blättern (Thymi herba) des Thymians (Thymus serpyllum L.)

10 kg getrocknete, gereinigte Thymianblätter werden in 80 Liter Wasser der Temperatur von 90 °C 15 Minuten lang gerührt. Dann wird das Gemisch filtriert und das Filtrat bis zur weiteren Verwendung vor Wärme, Licht und Luft geschützt aufbewahrt.

Beispiel 10

Herstellung eines alkoholischen Auszuges von Blättern (Thymi herba) des Thymians (Thymus serpyllum L.)

Man arbeitet auf die in Ph. Hg. VI beschriebene Weise.

Beispiel 11

4

Herstellung eines wäßrigen, kombinierten Drogenextraktes

Ein Gemisch aus Equiseti herba, Cynosbati pseudofructus und Graminis rhizoma im Verhältnis 1:5:1,5 wird in der siebenfachen Menge Wasser der Temperatur von 60 °Cdrei Tage lang eingeweicht. Dann wird das Gemisch filtriert und das Filtrat bis zur weiteren Verwendung vor Wärme, Licht und Luft geschützt gelagert.

Beispiel 12

Herstellung eines alkoholischen kombinierten Drogenextraktes

Ein im Verhältnis 1:2,5 bereitetes Gemisch aus Thymianblättern und Kamillenblüten wird zerkleinert und dann 24 Stunden lang in 90 %igem Äthanol eingeweicht. Die alkoholische Lösung wird filtriert und bis zur weiteren Verwendung vor Wärme, Licht und Luft geschützt gelagert.

Beispiel 13

Herstellung eines Fünfkomponenten-Wirkstoffes

Die gemäß den Beispielen 11 und 12 hergestellten Lösungen werden im Verhältnis 1:1 gemischt. Das Gemisch wird auf die in der kosmetischen Industrie üblichen Weise formuliert.

Beispiel 14

Herstellung·eines Trockenextraktes aus Graminis rhizoma

Der gemäß Beispiel 1 hergestellte wäßrige Extrakt wird unter aseptischen Bedingungen lyophilisiert. Das erhaltene Lyophilisat wird luftdicht abgeschlossen, vor Wärme und Licht geschützt aufbewahrt.

Beispiel 15

### Tageskrem

| | | |
|---|---|---|
| Wäßriger Extrakt von Graminis rhizoma | 28,0 | g |
| Cetylalkohol | 4,5 | g |
| Stearinsäure | 4,0 | g |
| BRIJ 721 | 4,0 | g |
| kosmetisches Vaselineöl | 1,5 | g |
| Ricinusöl | 2,0 | g |
| Propylenglycol | 5,0 | g |
| Retinoinsäure | 0,05 | g |
| Äthanol, 96 %ig | 5,0 | g |
| destilliertes Wasser | 45,55 | g |
| Stabilisator | 0,2 | g |
| Parfüm | 0,2 | g |

Beispiel 16

Nachtkrem

| | |
|---|---|
| alkoholischer Extrakt von Graminis rhizoma | 0,3 g |
| alkoholischer Extrakt von Thymian | 7,0 g |
| alkoholischer Extrakt von Kamillenblüten | 6,0 g |
| alkoholischer Extrakt von Hagebutten | 19,0 g |
| alkoholischer Extrakt von Schachtelhalm | 2,0 g |
| Ricinusöl | 3,0 g |
| Isopropylpalmitat | 5,0 g |
| Hostacerin CG | 10,0 g |
| Propylenglycol | 3,0 g |
| destilliertes Wasser, Konservierungsm. | 44,5 g |
| Parfüm | 0,2 g |

Beispiel 17

Krem

| | |
|---|---|
| Wäßriger Extrakt von Graminis rhizoma | 28,0 g |
| Cetylalkohol | 6,5 g |
| Stearin | 5,4 g |
| kosmetisches Vaselineöl | 1,5 g |
| Propylenglycol | 5,0 g |
| Sorbit | 3,0 g |
| Natriumlaurylsulfat | 0,5 g |
| Nipagin M | 0,2 g |

| | |
|---|---|
| Erythromycin (freie Base) | 1,5 g |
| Äthanol, 96 %ig | 4,0 g |
| destilliertes Wasser, Konservierungsmittel | 44,2 g |
| Parfüm | 0,2 g |

Beispiel 18

Waschemulsion

| | |
|---|---|
| Wäßriger Extrakt von Grminis rhizoma | 28,0 g |
| wäßriger Extrakt von Kamillenblüten | 2,0 g |
| wäßriger Extrakt von Hagebutten | 5,0 g |
| Hostaphat KL 340 N | 2,0 g |
| Hostacerin DGS | 4,0 g |
| Hostacerin PN 73 | 0,6 g |
| Paraffinöl | 5,0 g |
| Isopropylpalmitat | 6,0 g |
| Propylenglycol | 3,0 g |
| destilliertes Wasser, Konservierungsmittel | 44,2 g |
| Parfüm | 0,2 g |

Beispiel 19

Tonikum

| | |
|---|---|
| Alkoholischer Extrakt von Graminis rhizoma | 28,0 g |
| Erythromycin (freie Base) | 1,5 g |
| Äthanol, 96 %ig | 48,5 g |
| Propylenglycol | 5,0 g |
| destilliertes Wasser | 16,8 g |
| Parfüm | 0,2 g |

Beispiel 20

Tonikum

| | |
|---|---|
| Alkoholischer Extrakt von Graminis rhizoma | 28,0 g |
| Retinoinsäure | 0,05 g |
| Äthanol, 96 %ig | 61,25 g |
| Propylenglycol | 10,0 g |
| Tween 20 | 0,5 g |
| Parfüm | 0,2 g |

Beispiel 21

7

Seife

| Trockenextrakt von Graminis rhizoma | 2,0 g |
|---|---|
| Seifenspäne | 60,0 g |
| Hostapon T | 10,0 g |
| Natriumtripolyphosphat | 10,0 g |
| Natriumcitrat | 13,0 g |

Beispiel 22

Flüssige Seife

| Wäßriger Extrakt von Graminis rhizoma | 30,0 g |
|---|---|
| wäßriger Extrakt von Schachtelhalm | 2,0 g |
| wäßriger Extrakt von Hagebutten | 5,0 g |
| Genapol TSM | 3,0 g |
| Genapol LRO | 35,0 g |
| Medilan KA | 8,0 g |
| Natriumchlorid | 1,3 g |
| destilliertes Wasser, Konservierungsmittel | 15,5 g |
| Parfüm | 0,2 g |

Beispiel 23

Shampoon

| Wäßriger Extrakt von Graminis rhizoma | 24,0 g |
|---|---|
| wäßriger Extrakt von Thymian | 4,0 g |
| wäßriger Extrakt von Hagebutten | 6,0 g |
| wäßriger Extrakt von Kamillenblüten | 3,0 g |
| Genapol ZRO | 35,0 g |
| Genapol AMS | 10,0 g |
| Betain | 8,0 g |
| Kokusfettsäurediäthanolamid | 2,0 g |
| destilliertes Wasser, Konservierungsmittel | 6,4 g |
| Natriumchlorid | 1,4 |
| Parfüm | 0,2 g |

Beispiel 24

Gelee für Gesichtspackungen

| | |
|---|---|
| wäßriger Extrakt von Graminis rhizoma | 25,0 g |
| wäßriger Extrakt von Schachtelhalm | 10,0 g |
| wäßriger Extrakt von Hagebutten | 5,0 g |
| Carbopol 940 | 1,0 g |
| Natronlauge, 10 %ig | 2,0 g |
| destilliertes Wasser, Konservierungsmittel | 56,8 g |
| Parfüm | 0,2 g |

Beispiel 25

Tonikum

| | |
|---|---|
| Wäßriger Extrakt von Graminis rhizoma | 28,0 g |
| Erythromycin-lactobionat | 2,25g |
| Propylenglycol | 10,0 g |
| Äthanol 96 %ig | 43,5 g |
| ionenfreies Wasser | 16,25g |

Beispiel 26

Tageskrem

| | |
|---|---|
| Wäßriger Auszug von Graminis rhizoma | 28,0 g |
| Erythromycin-lactobionat | 2,25 g |
| Cetylalkohol | 10,0 g |
| BRIJ 721 | 4,0 g |
| Carbopol 940 | 0,10 g |
| Nipagin M | 0,20 g |
| ionenfreies Wasser | 55,45 g |

Die in den Beispielen beschriebenen Präparate wurden einer klinischen Untersuchung unterzogen. In die Untersuchung wurden 69 Personen (43 Frauen, 26 Männer) einbezogen. Das Lebensalter lag zwischen 12 und 30 Jahren, das Durchschnittslebensalter bei 16 Jahren. Die Kranken verteilten sich auf die unterschiedlichen Schweregrade der Akne wie folgt: Acne comedonica mit schwereren Symptomen: 55, daneben ausgedehnte Papulopustolose bei 25 und Acne conglobata bei 3 Kranken.

Die Schwere des Krankheitsprozesses wurde auf Grund der auf einer Wange befindlichen nicht-entzündeten (offene und geschlossene Komedonen) und entzündete Erscheinungen (Papulen, Pusteln) in vier Grade eingeteilt.

I        weniger als 10

9

II     11-25

III    26-50

IV    mehr als 50

Die Anzahl der behandelten Personen, die Schwere des klinischen Befundes und die mit den Präparaten erzielten Ergebnisse wurden in der Tabelle 1 gesammelt (Personen, die gleichzeitig an verschiedenen Erscheinungsformen der Akne leiden, erscheinen in der Tabelle bei jeder Erscheinungsform einmal). Außerdem wurden die in Form subjektiver und objektiver Beschwerden auftretenden Nebenwirkungen (Brennen, Jucken, Hautröte, Schälen) ausgewertet.

Bei Behandlung mit dem Präparat gemäß Beispiel 15 konnten die Fälle mit dem Schweregrad II und III (zum überwiegenden Teil Acne comedonica) in einer Zeit von 8-12 Wochen symptomfrei gemacht werden oder zeigten doch zumindest eine wesentliche Besserung. Bei Kombination der verschiedenen Aknearten war weitere kontinuierliche Behandlung erforderlich. Die Kranken, die Komedomenbildung und Papulopsteln der Stufe IV und außerdem noch Acne conglobata aufwiesen, mußten länger als 12 Wochen behandelt werden.

Die Krem gemäß Beispiel 16 heilt bei täglich zweimaliger Anwendung die schwach entzündlichen Symptome (diffuses Erythema, wenige Papulen) im allgemeinen innerhalb von ein bis zwei Wochen. Die Kram ist für vorbeugende Zwecke sowie für die Nachbehandlung geeignet. Keine der behandelten 13 Personen beklagte sich über irritative Nebenwirkungen oder Intoleranz.

An 13 Kranken wurde unter Einbeziehung von 8 Kontrollpersonen ein Foto-patch Test durchgeführt. Das Lebensalter der Kranken betrug durchschnittlich 13,5 Jahre (12-18 Jahre), von den Personen waren 2 männlichen, 11 weiblichen Geschlechts. 9 der Kranken litten an Acne comedonica, 4 an Acna populopustulosa. Die epicutan aufgetragene Substanz wurde nach 24 Stunden entfernt. Dann wurden die getesteten sowie zur Kontrolle die gegenüberliegenden Gliedmaßen für je 20 Minuten mit 5 J/cm$^2$ Lichtenergie bestrahlt. Es konnte festgestellt werden, daß sich beim Foto-patch Test weder die 13 Kranken noch die Kontrollpersonen über irritative Wirkungen beziehungsweise frühe oder späte Allergiereaktionen zu beklagen hatten.

Das Präparat gemäß Beispiel 25 wurde an 9 Kranken ausprobiert. Als Ort der lokalen Anwendung kamen hauptsächlich das Gesicht, in manchen Fällen auch der Rücken, die Schultern und der Brustkorb in Frage. Nach einer gründlichen Reinigung der zu behandelnden Flächen mit warmem Seifenwasser wurde die Haut zu Beginn täglich zweimal, nach 10 Tagen täglich nur noch einmal aufgetragen. In allen Fällen trat Besserung ein; diese war bei den schweren Formen der Akna am augenfälligsten. Bei seborrhischer, fettiger Haut ging die Röte des Gesichtes zurück, das Hautjucken wurde schwächer und die Entzündungen verschwanden. Bei schweren Fällen von Papulopusteln wurden die subjektiven Beschwerden (Brennen, Jucken) häufig schwächer. Bei kontinuierlicher Behandlung nimmt die Zahl der Papulen ständig ab, der fettige Glanz und die Komedomenbildung mäßigen sich.

Tabelle 1

| Komposition gemäß Beispiel | Anzahl der Personen männl. | weibl. | Acne comedonica I | II | III | IV | Acne pop. pustulosa I | II | III | IV | conglobata I | II | III | IV | Ergebnisse geheilt | gebessert | unverändert |
|---|---|---|---|---|---|---|---|---|---|---|---|---|---|---|---|---|---|
| 15 | 8 | 5 | | 2 | 4 | 6 | 1 | 8 | 2 | 1 | 2 | | | | 2 | 11 | - |
| 17 | 11 | 6 | | 2 | 11 | 4 | 1 | 6 | 8 | 2 | | | | | 5 | 12 | - |
| 18 | 11 | 1 | 1 | 6 | 4 | | 2 | 7 | | | | 1 | | | 6 | 6 | - |
| 19 | 5 | 9 | | | 9 | 5 | | 5 | 8 | 1 | | | | | 1 | 13 | |
| 20 | 8 | 5 | | 1 | 9 | 3 | 1 | 6 | 3 | | | | | | 6 | 7 | |
| 25 | 7 | 2 | 2 | 1 | 2 | | 1 | 1 | 2 | | | | | | 3 | 6 | |
| insgesamt | 50 | 28 | 3 | 12 | 39 | 18 | 6 | 33 | 23 | 4 | 3 | | | | 23 | 55 | |

1. Präparate mit Wirkung gegen die verschiedenen Formen der Akne, insbesondere in Form von Creme, Tonikum, Seife, Körperpflegelotionen, Gesichtspackungen und Shampoos, dadurch **gekennzeichnet,** daß sie 2-95 Gewichtsprozent saponin-haltigen Wirkstoffextrakt aus dem Wurzelstock (Graminis rhizoma) des Queckenweizens (Agropyron repens) und 5-98 Gewichtsprozent der in der Kosmetik im allgemeinen üblichen Zusätze und/oder Verdünnungsmittel, vorzugsweise natürliche Fette, Öle, Emulgiermittel, Konservierungsmittel, Duftstoffe und gegebenenfalls ein Antibiotikum und/oder weitere Pflanzenextrakte enthalten.

2. Präparat nach Anspruch 1, dadurch **gekennzeichnet,** daß es 0,2-6,0 Gew.-% Antibiotikum, vorzugsweise Erythromycin, insbesondere in der Form von Erythromycin-lactobionat, enthält.

3. Präparat nach Anspruch 1, dadurch **gekennzeichnet,** daß es als weiteren Pflanzenextrakt Extrakt aus Hagebutten, Schachtelhalm, Thymian und/oder Kamille enthält.

4. Verwendung von saponin-haltigem Wirkstoffextrakt aus dem Wurzelstock des Queckenweizens (Agropyron repens) zur Herstellung von Präparaten gegen Akne.

**Patentansprüche für folgenden Vertragsstaat: AT**

1. Präparat mit Wirkung gegen die verschiedenen Formen der Akne, insbesondere in Form von Creme, Tonikum, Seife, Körperpflegelotionen, Gesichtspackungen und Shampoos, dadurch **gekennzeichnet,** daß sie 2-95 Gewichtsprozent saponin-haltigen Wirkstoffextrakt aus dem Wurzelstock (Graminis rhizoma) des Queckenweizens (Agropyron repens) und 5-98 Gewichtsprozent der in der Kosmetik im allgemeinen üblichen Zusätze und/oder Verdünnungsmittel, vorzugsweise natürliche Fette, Öle, Emulgiermittel, Konservierungsmittel, Duftstoffe und gegebenenfalls ein Antibiotikum und/oder weitere Pflanzenextrakte enthalten.

2. Präparat nach Anspruch 1, dadurch **gekennzeichnet,** daß es 0,2-6,0 Gew.-% Antibiotikum, vorzugsweise Erythromycin, insbesondere in der Form von Erythromycin-lactobionat, enthält.

3. Präparat nach Anspruch 1, dadurch **gekennzeichnet,** daß es als weiteren Pflanzenextrakt Extrakt aus Hagebutten, Schachtelhalm, Thymian und/oder Kamille enthält.

4. Verwendung von saponin-haltigem Wirkstoffextrakt aus dem Wurzelstock des Queckenweizens (Agropyron repens) zur Herstellung von Präparaten gegen Akne.

**Claims**
**Claims for the following Contracting States: BE, CH, DE, FR, GB, IT, LI, LU, NL, SE**

1. Preparations having an action against the various forms of acne, especially in the form of a cream, tonic, soap, body lotions, face packs and shampoos, **characterised in that** they contain from 2 to 95 % by weight saponin-containing active ingredient extract from the rootstock (Graminis rhizoma) of couch grass (Agropyron repens) and from 5 to 98 % by weight additives and/or diluents generally customary in cosmetics, preferably natural fats, oils, emulsifying agents, preservatives, perfumes and optionally an antibiotic and/or further plant extracts.

2. Preparation according to claim 1, **characterised in that** it contains from 0.2 to 6.0 % by weight antibiotic, preferably erythromycin, especially in the form of erythromycin lactobionate.

3. Preparation according to claim 1, **characterised in that** as further plant extract it contains extract of rose-hips, horsetail, thyme and/or camomile.

4. The use of a saponin-containing active ingredient extract from the rootstock of couch grass (Agropyron repens) for the manufacture of preparations against acne.

**Claims for the following Contracting State: AT**

1. Preparations having an action against the various forms of acne, especially in the form of a cream, tonic, soap, body lotions, face packs and shampoos, **characterised in that** they contain from 2 to 95 % by weight saponin-containing active ingredient extract from the rootstock (Graminis rhizoma) of couch grass (Agropyron repens) and from 5 to 98 % by weight additives and/or diluents generally customary in cosmetics, preferably natural fats, oils, emulsifying agents, preservatives, perfumes and optionally an antibiotic and/or further plant extracts.

2. Preparation according to claim 1, **characterised in that** it contains from 0.2 to 6.0 % by weight antibiotic, preferably erythromycin, especially in the form of erythromycin lactobionate.

3. Preparation according to claim 1, **characterised in that** as further plant extract it contains extract of rose-hips, horsetail, thyme and/or camomile.

4. The use of a saponin-containing active ingredient extract from the rootstock of couch grass (Agropyron repens) for the manufacture of preparations against acne.

**Revendications**
**Revendications pour les Etats contractants suivants: BE, CH, DE, FR, GB, IT, LI, LU, NL, SE**

1. Préparations agissant contre les différentes formes d'acné, en particulier sous forme de crème, de tonique, de savon, de lotions pour soins corporels, de masques faciaux et de shampooings, caractérisées en ce qu'elles contiennent 2 à 95 % en poids d'extrait actif, contenant de la saponine, du rhizome (Graminis rhizoma) de chiendent (Agropyron repens) et 5 à 98 % en poids d'adjuvants et/ou d'excipients habituellement utilisés en cosmétique, de préférence des graisses naturelles, des huiles, des émulsifiants, des agents de conservation, des arômes et éventuellement un antibiotique et/ou d'autres extraits végétaux.

2. Préparation selon la revendication 1, caractérisée en ce qu'elle contient 0,2 à 0,6 % en poids d'antibiotique, de préférence l'érythromycine, en particulier sous forme de lactobionate d'érythromycine.

3. Préparation selon la revendication 1, caractérisée en ce qu'elle contient comme extrait végétal supplémentaire un extrait de fruits d'églantier, de prêle, de thym et/ou de camomille.

4. Utilisation d'extrait actif, contenant de la saponine, du rhizome de chiendent (Agropyron repens) pour la fabrication de préparations contre l'acné.

**Revendications pour l'Etat contractant suivant: AT**

1. Préparations agissant contre les différentes formes d'acné, en particulier sous forme de crème, de tonique, de savon, de lotions pour soins corporels, de masques faciaux et de shampooings, caractérisées en ce qu'elles contiennent 2 à 95 % en poids d'extrait actif, contenant de la saponine, du rhizome (Graminis rhizoma) de chiendent (Agropyron repens) et 5 à 98 % en poids d'adjuvants et/ou d'excipients habituellement utilisés en cosmétique, de préférence des graisses naturelles, des huiles, des émulsifiants, des agents de conservation, des arômes et éventuellement un antibiotique et/ou d'autres extraits végétaux.

2. Préparation selon la revendication 1, caractérisée en ce qu'elle contient 0,2 à 0,6 % en poids d'antibiotique, de préférence l'érythromycine, en particulier sous forme de lactobionate d'érythromycine.

3. Préparation selon la revendication 1, caractérisée en ce qu'elle contient comme extrait végétal supplémentaire un extrait de fruits d'églantier, de prêle, de thym et/ou de camomille.

4. Utilisation d'extrait actif, contenant de la saponine, du rhizome de chiendent (Agropyron repens) pour la fabrication de préparations contre l'acné.